(19) Europäisches Patentamt
European Patent Office
Office européen des brevets

(11) Veröffentlichungsnummer: 0 101 886
A2

(12) # EUROPÄISCHE PATENTANMELDUNG

(21) Anmeldenummer: 83107087.5

(22) Anmeldetag: 20.07.83

(51) Int. Cl.³: C 07 G 7/00
G 01 N 33/68, G 01 N 33/56
G 01 N 33/54, G 01 N 33/58

(30) Priorität: 21.08.82 DE 3231204

(43) Veröffentlichungstag der Anmeldung:
07.03.84 Patentblatt 84/10

(84) Benannte Vertragsstaaten:
AT BE CH DE FR GB IT LI LU NL SE

(71) Anmelder: BOEHRINGER INGELHEIM DIAGNOSTIKA GMBH
Gutenbergstrasse 3 Postfach 1609
D-8046 Garching bei München(DE)

(72) Erfinder: Kolb, Helmut, Prof. Dr.
Kranzhornstrasse 13
D-8000 München 82(DE)

(72) Erfinder: Schleifer, Karl Heinz, Prof. Dr.
Schwalbenstrasse 3a
D-8044 Lohhof(DE)

(72) Erfinder: Seidl, Hans Peter, Dr.
Marsopstrasse 20
D-8000 München 60(DE)

(72) Erfinder: Tympner, Klaus Dieter, Prof. Dr.
Waterloostrasse 70
D-8000 München 71(DE)

(72) Erfinder: Weiss, Ludwig, Dr.
Hauberrisser Strasse 7
D-8000 München 90(DE)

(54) **Antikörper gegen bakterielle Peptidoglycane, Verfahren zu ihrer Herstellung und Methoden zu ihrer quantitativen Bestimmung.**

(57) Antikörper aus der Klasse der Immunglobuline G gegen das Peptiduntereinheit-Pentapeptid L-Ala-D-Glu(L-Lys-D-Ala-D-Ala) des Peptidoglycans in biologischem Untersuchungsmaterial sowie gegen diese Peptidoglycane enthaltende bakterielle Zellwände, gekennzeichnet durch

a) spezifische Bindung an Pentapeptide der allgemeinen Formel

$$R - A_1 - D - Ala \qquad (I)$$

worin

R ein Aminosäureanteil mit 1 – 3, vorzugsweise 3 Aminosäuren aus der Gruppe Gly, L-Ala, D-Glu, L-Lys und

$A_1$ eine der Aminosäuren D-Ala, D-Ser, D-Val oder D-Leu bedeuten, oder

b) spezifische Bindung an Pentapeptide der allgemeinen Formel I enthaltende isolierte bakterielle Zellwände oder

c) spezifische Bindung an Pentapeptide der allgemeinen Formel I enthaltende grampositive Bakterien,

Verfahren zu ihrer Herstellung, Verfahren zu ihrer quantitativen Bestimmung und Verwendung.

EP 0 101 886 A2

Unter dem Überbegriff "Kollagenosen" werden heute eine Gruppe von Erkrankungen, die sich in generalisierten Formen im Bindegewebe abspielen, wie z.B. das rheumatische Fieber, die chronische Polyarthritis, der Lupus erythematodes, die Arteriitis temporalis Horton und viele andere zusammengefaßt.
Manche dieser Erkrankungen, die auch als Autoimmunoerkrankungen bezeichnet werden, lassen sich aufgrund ihres klinischen Erscheinungsbildes oft schwer voneinander unterscheiden. Außerdem können bei ein und demselben Patienten unterschiedliche Bilder der verschiedenen Ausprägungen einer Kollagenose vorkommen.

Unter den oben angeführten Erkrankungen stellt das rheumatische Fieber einen Sonderfall dar, denn für seine Entstehung gilt eine vorhergehende Infektion mit hämolysierenden Streptokokken der Gruppe A als der ätiologische Faktor. Bei diesen Patienten ergeben die sogenannten "Rheumateste" im Serum in einem hohen Maß (80-90 %) eine positive Reaktion. Hierbei handelt es sich um Antikörper gegen intrazelluläre Bestandteile der Streptokokken wie Streptolysis O, Desoxyribonuclease, Hyaluronidase etc..

Die chronische Polyarthritis (rheumatoide Arthritis) dagegen ist die häufigste Kollagenose und führt zu einer wesentlichen Beeinträchtigung der Gesundheit. Heute nimmt man eine Erkrankungshäufigkeit von mindestens 10 % der Gesamtbevölkerung an.
Das Ergebnis der "Rheumateste" ist jedoch nur bei etwa der Hälfte der Patienten positiv. Dies gilt in ganz besonderem Maße für das Kindesalter.

Der Schwerpunkt der bisherigen Untersuchungen hinsichtlich der Pathogenese der rheumatoiden Arthritis und auch der Diagnostik dieser Erkrankung lag im wesentlichen in der Vorstellung, daß intrazelluläre Bestandteile der Bakterien als Antigen wirksam werden, zur Antikörperproduktion anregen und unter Bildung von Immunkomplexen im Bindegewebe chronisch entzündliche Reaktionen auslösen.

Untersuchungen in neuerer Zeit weisen jedoch darauf hin, daß der bakteriellen Zellwand und insbesondere den Peptidoglycanen bei der Induktion chronischer Entzündungsprozesse mit oder ohne initial (bakteriologisch) nachweisbarer Infektion eine weit größere Bedeutung für die persistierende Immunantwort zukommt.

Peptidoglycane (synonym: Mureine, Mucopeptide) stellen die wichtigste Komponente der bakteriellen Zellwand dar und sind bei allen Bakterien vorhanden. Pilze, Rickettsien und Viren besitzen dagegen keine entsprechenden Komponenten.

Tierexperimentell wurde nachgewiesen, daß Peptidoglycane im Säugetierorganismus neben der Induktion akuter Entzündungsprozesse ausgesprochen chronische Entzündungen hervorrufen können. Darüber hinaus zeigten Untersuchungen, daß bakterielle Zellwände aufgrund ihrer weitgehenden Resistenz gegenüber allen bekannten animalischen Enzymen über Monate bis Jahre in Geweben lokalisiert bleiben.
Es wurde daher vermutet, daß solche Erregerkomponenten möglicherweise bei der Entstehung primär chronischer Entzündungen bisher unbekannter Ätiologie (z.B. der chronischen Polyarthritis) eine Rolle spielen.

Peptidoglycane sind also praktisch ubiquitär vorkommende hochpotente Antigene, gegen die im animalischen Organismus häufig Antikörper gebildet werden.

Es ist bekannt, daß das besonders bei Streptokokken und Staphylokokken vorkommende, nicht an der Quervernetzung beteiligte Pentapeptid der Struktur L-Ala-D-Glu(L-Lys-D-Ala-D-Ala) eine wesentliche antigene Determinante des Peptidoglycans darstellt. Dieses Peptid wird nachfolgend als "Peptiduntereinheit-Pentapeptid" bezeichnet. Die C-terminale Sequenz -D-Ala-D-Ala des Peptiduntereinheit-Pentapeptids ist als "immundominante Gruppe" von ausschlaggebender Bedeutung für die Bindung spezifischer Antikörper an die antigene Determinante L-Ala-D-Glu(L-Lys-D-Ala-D-Ala).

Spezifische Antikörper gegen dieses Peptiduntereinheit-Pentapeptid sind daher von ganz besonderem diagnostischen Interesse für die Erkennung und Therapieüberwachung von "Autoimmunerkrankungen" (wie z.B. der chronischen Polyarthritis); ihre Anwesenheit läßt auf Streptokokken bzw. Staphylokokken als ätiologische Faktoren schließen.

Bisher wurde unter Einsatz verschiedener Techniken, wie z.B. der Immunpräzipitation das Vorkommen von Antikörpern gegen den Peptidoglycan-Anteil bakterieller Zellwände in tierischen Seren beschrieben. Zum Nachweis von Antikörpern gegen das Peptiduntereinheit-Pentapeptid des Peptidoglycans in tierischen Seren ist ein Hapten-Bindungs-Assay beschrieben (J. Immunol. 114, 1191-1196 (1975)). Das veröffentlichte Verfahren erlaubt jedoch nur eine grob quantitative Abschätzung des Antikörpergehalts in menschlichen Seren, da die Quantifizierung über Kaninchenhyperimmunseren erfolgt.

Diese Methode weist verschiedene Nachteile auf:

1. Nicht-präzipitierbare Antikörper sind damit nicht bestimmbar. Die Autoren selbst geben eine starke Abweichung des mit unterschiedlichen Methoden bestimmten Antikörpergehalts an (z.B. spezifischer Antikörpergehalt, bestimmt mit dem Hapten-Bindungs-Assay 4.6 mg/ml; spezifischer Antikörpergehalt, bestimmt nach Präzipitationsverfahren 2.2 mg/ml).

2. Der Nachweis, daß es sich bei dem präzipitierten Protein um Immunglobuline handle, erfolgt nicht. Eine Kopräzipitation von Nicht-Immunglobulinen ist daher nicht auszuschließen.

3. Aus einer Vielzahl immunologischer Studien ist bekannt, daß sich tierische Antikörper mit gegen das gleiche Antigen gerichteten menschlichen Antikörpern hinsichtlich ihrer Affinität nicht vergleichen lassen.

Hinsichtlich des Hapten-Bindungstestes selbst ist einzuwenden, daß in der Form, wie der Test durchgeführt wurde, keine Differenzierung der Immunglobulinklassen erfolgen kann.

Darüber hinaus ist der Hapten-Bindungstest in der beschriebenen Form durch im Untersuchungsmaterial vorkommende Substanzen leicht zu stören, wie z.B. durch die Gegenwart von freiem Antigen oder durch strukturähnliche Antibiotika. Diese hohe Störanfälligkeit ergibt sich aus der Tatsache, daß solche Substanzen die Gleichgewichtslage der löslichen Antikörper-Haptenkomplexe sehr leicht

beeinflussen können.

Gegen die Verwendung des Hapten-Bindungstestes als Routinemethode zum Nachweis von Antikörpern gegen Peptidoglycan spricht jedoch hauptsächlich, daß neben $^{125}J$ zur Markierung des Antigens $^{22}Na$ als interner Standard Verwendung findet. Die Verwendung eines zweiten, in nicht unbeträchtlicher Radioaktivität eingesetzten Isotops ($^{22}Na$, ein harter ß-Strahler), kann zwar in Ausnahmefällen bei wissenschaftlichen Untersuchungen berechtigt sein, keinesfalls ist eine derartige Methode jedoch für Routineuntersuchungen zu vertreten.

Die Erfindung betrifft Antikörper aus der Klasse der Immunglobuline G gegen das Peptiduntereinheit-Pentapeptid L-Ala-D-Glu-(L-Lys-D-Ala-D-Ala) des Peptidoglycans sowie gegen dieses Peptidoglycan enthaltende bakterielle Zellwände, Verfahren zu ihrer Herstellung und Methoden zu ihrer quantitativen Bestimmung in biologischem Untersuchungsmaterial; die Bestimmungsmethoden können für Routineuntersuchungen verwendet werden.

Die Antikörper sind charakterisiert

a) durch spezielle Bindung an Pentapeptide der allgemeinen Formel

$$R - A_1 - D - Ala \qquad (I)$$

worin

R ein Aminosäureanteil mit 1 - 3, vorzugsweise 3 Aminosäuren aus der Gruppe Gly, L-Ala, D-Glu, L-Lys und

$A_1$ eine der Aminosäuren D-Ala, D-Ser, D-Val oder D-Leu bedeuten; oder

b) durch Bindung an isolierte bakterielle Zellwände, die diese Pentapeptide enthalten; oder

c) durch Bindung an grampositive Bakterien, die diese Pentapeptide enthalten.

Die Erfindung betrifft ferner ein Verfahren zur Herstellung der Antikörper gegen das Peptiduntereinheit-Pentapeptid L-Ala-D-Glu (L-Lys-D-Ala-D-Ala) des Peptidoglycans sowie gegen diese Pentapeptide enthaltende Zellwände.

Die Erfindung betrifft außerdem Verfahren zur quantitativen Bestimmung von Antikörpern gegen das Peptiduntereinheit-Pentapeptid L-Ala-D-Glu (L-Lys-D-Ala-D-Ala) des Peptidoglycans in biologischem Untersuchungsmaterial; als solche Verfahren seien genannt der Doppel-Antikörper-Radioimmun-Assay, der Doppel-Antikörper-Enzymimmun-Assay, die nephelometrische Methode, der Festphasen-Sandwich-Radioimmun-Assay, der Festphasen-Enzymimmun-Assay, der Festphasen-Fluoreszenzimmun-Assay sowie der Latex-Agglutinationstest.

b) Bestimmung des Antikörpers:

1. Standardgewinnung

Standardlösungen von Antikörpern mit Spezifität gegen das Peptiduntereinheit-Pentapeptid L-Ala-D-Glu(L-Lys-D-Ala-D-Ala) werden durch Affinitätschromatographie (mit Matrix gebundenen Peptiden der Sequenz-R-D-Ala-D-Ala) aus Patientenseren, in denen solche Antikörper nachgewiesen wurden, gewonnen. Nach Bindung der spezifischen Antikörper an die Affinitäts-Matrix wird nichtgebundenes Serumprotein durch Waschen mit Pufferlösungen entfernt. Die Elution der an die feste Matrix gebundenen Antikörper erfolgt durch spezifische Desorption der gebundenen Immunglobuline mit Peptiden der Sequenz R-D-Ala-D-Ala. Die Desorption kann ebenfalls durch im Prinzip veröffentlichte Techniken wie Anwendung tiefer pH-Werte oder hoher Salzkonzentrationen erfolgen. Nach Dialyse gegen Pufferlösungen wird der Gehalt an spezifischen Antikörpern gegen das Peptiduntereinheit-Pentapeptid des Peptidoglycans über den Immunglobulingehalt der gereinigten Fraktion quantifiziert.

2. Testaufbau

Die quantitative Bestimmung von spezifischen Antikörpern der verschiedenen Immunglobulinklassen gegen das Peptiduntereinheit-Pentapeptid des Peptidoglyans läßt sich mit zwei prinzipiell verschiedenen Methoden durchführen.

a) Quantitative Bestimmung im löslichen System (Messung in homogener Phase)

b) Quantitative Bestimmung durch Messung im heterogenen System (Messung in heterogener Phase; Festphasen-Immuno-Assay).

2.1. Messung in der homogenen Phase, z.B.

2.1.1. quantitative Bestimmung von Antikörpern gegen das Peptiduntereinheit-Pentapeptid des Peptidoglycans in einem Doppel-Antikörper-Radioimmuno-Assay.

Peptide der Sequenz R-D-Ala-D-Ala werden radioaktiv markiert und der Testansatz entsprechend nachfolgendem Schema ausgeführt:

$$*\text{Pept.} + \text{Ak} \longrightarrow *\text{Pept.-Ak}$$

Die Trennung von radioaktivem freien Antigen von radioaktivem Antikörper-gebundenem Antigen erfolgt nach der Doppel-Antikörpermethode durch spezifische Präzipitation der Immunglobuline mit Hilfe eines zweiten Anti-Antikörpers nach folgendem Schema:

$$*\text{Pept.-Ak} + \text{Anti-Ak} \longrightarrow *\text{Pept.-Ak}_m\text{-(Anti-Ak)}_m\text{-Komplexe}$$

Die radioaktive Markierung des Antigens kann z.B. erfolgen

a) durch Kopplung von Tyrosin an das N-terminale Ende des Peptids und anschließende Jodierung mit $^{125}J$ mit konventionellen Methoden

b) durch Jodierung mit (N-Succinimidyl-3-(4-Hydroxy, 5-($^{125}J$)Jodophenyl)propionat

c) durch Tritierung des Antigens mit N-Succinimidyl (2,3-$^{3}H$)propionat.

Durch selektive Auswahl der Spezifität des zweiten Antikörpers lassen sich separat verschiedene Immunglobulinklassen, wie z.B. IgG oder IgM etc. bestimmen.

Es empfiehlt sich, die verwendeten Anti-Antikörper vor Verwendung durch Affinitätschromatographie an Matrix gebundenen Peptiden der Sequenz R-D-Ala-D-Ala zur Entfernung von eventuell enthaltenden Antikörpern gegen Peptidoglycan zu reinigen.

2.1.2. Quantitative Bestimmung von Antikörpern gegen das Peptiduntereinheit-Pentapeptid in einem Doppelantikörper-Enzymimmuno-Assay

Testaufbau wie unter 2.1.1., jedoch erfolgt die Markierung der Anti-Antikörper mittels eines Enzyms wie z.B. alkalische Phosphatase, Peroxidase, Glucose-Oxidase-Peroxidase, Glucose-6-Phosphatdehydrogenase, Malat-Dehydrogenase etc. anstelle von Radioaktivität.

2.1.3. Quantitative Bestimmung von Antikörpern gegen das Peptiduntereinheit-Pentapeptid des Peptidoglycans mittels nephelometrischer Methoden.
Peptide der Sequenz R-D-Ala-D-Ala werden über die N-terminale Aminogruppe an eine lösliche Matrix, z.B. Proteine, Polyvinyl-Pyrrolidon etc. gebunden und der Testansatz entsprechend nachfolgendem Schema ausgeführt:

$$\text{Matrix-(Pept.)}_n + \text{Ak} \longrightarrow \text{Matrix-(Pept.)}_n\text{-Ak-Komplexe}$$

Trübung

Die Kopplung der Peptide an das Trägermaterial kann z.B. erfolgen

1. durch Bindung der jodacetylierten Peptide an die Matrix
2. mit der Carbodiimid-Methode
3. mittels bifunktioneller Reagenzien, z.B. Diisocyanate, Glutardialdehyd etc..

2.2. Messung in der heterogenen Phase

Peptide der Sequenz R-D-Ala-D-Ala werden über ihre N-terminale Aminogruppe an eine feste, unlösliche Matrix gebunden, wie z.B. Papier, Kunststoffe, Latex etc.. Die Kopplung erfolgt nach konventionellen Methoden. Die Bindung der Peptide kann entweder direkt an die Matrix erfolgen oder über sogenannte "spacer". Als "spacer" können dienen: aliphatische Kohlenwasserstoffketten, Proteine wie z.B. Albumin, RNase etc..

2.2.1. Quantitative Bestimmung von Antikörpern gegen das Peptiduntereinheit-Pentapeptid des Peptidoglycans in einem Festphasen-"Sandwich"-Radio-Immuno-Assay

2.2.2. Quantitative Bestimmung von Antikörpern gegen das Peptiduntereinheit-Pentapeptid des Peptidoglycans in einem Festphasen-Enzym-Immuno-Assay.

Testaufbau wie unter 2.2.1., jedoch erfolgt die Markierung der Anti-Antikörper mittels eines Enzyms wie unter 2.1.2. aufgeführt, anstelle von Radioaktivität.

2.2.3. Quantitative Bestimmung von Antikörpern gegen das Peptiduntereinheit-Pentapeptid des Peptidoglyans in einem Festphasen-Fluoreszenz-Immuno-Assay.

Testaufbau wie unter 2.2.1., jedoch erfolgt die Markierung des Antikörpers mittels eines Fluoreszenz-Farbstoffes (vgl. auch FIAX® - StiQ™-System).

2.2.4. Quantitative Bestimmung von Antikörpern gegen das Peptiduntereinheit-Pentapeptid des Peptidoglycans mittels eines Latex-Agglutinationstestes.

Peptide der Sequenz R-D-Ala-D-Ala werden entweder direkt oder über "spacer", wie z.B.Proteine, an Latexpartikel gekoppelt.

Bei den unter 2.1. sowie unter 2.2. aufgeführten quantitativen Bestimmungemethoden erfolgt die Quantifizierung mittels Eichkurve, die mit Hilfe der unter 1. beschriebenen Antikörperstandardlösungen aufgestellt werden kann.

Beispiel zur Bestimmung von Antikörpern der Klasse IgG gegen Pentapeptid.

1. Herstellung des Konjugats

Äquimolare Mengen von Peptiden mit der C-terminalen Aminosäure D-Ala der Sequenz R-D-Ala-D-Ala, vorzugsweise D-Ala-D-Ala-D-Ala und Jodacetylsuccinimidester in Dioxan: Wasser 1:2 (v:v), enthaltend 100 mmol/l Natriumhydrogencarbonat werden bei $4^{o}$ C 20 Stunden unter leichtem Rühren inkubiert. Das Dioxan wird anschließend im Vakuum abgezogen und das gebildete Reaktionsprodukt aus der wässrigen Phase durch Ansäuern mit HCl ausgefällt. Nach Waschen in salzsaurem Milieu wird das Produkt lyophilisiert.

100 mg jodacetyliertes Peptid und Trägerprotein, z.B. 250 mg menschliches Serumalbumin werden in 8 molarem Harnstoff, enthaltend 0,1 mM Natriumbicarbonat, 72 Stunden bei $37^{o}$C inkubiert. Nach 3-tägiger Dialyse gegen destilliertes Wasser (Wechsel der Dialysierflüssigkeit 6 mal) wird das an Albumin gekoppelte Peptid lyophilisiert. Nach dem beschirebenen Verfahren werden im Mittel 10 Mol Peptid pro Mol Trägerprotein kovalent gebunden.

2. Adsorption des Konjugats an Kunststoffoberflächen, z.B. Polystyrol

Im vorliegenden Beispiel wurden Polystyrolröhrchen mit einem Fassungsvermögen von knapp 2 ml, 4 cm hoch, Durchmesser: 1 cm, verwandt. Zur Bindung eignen sich such z.B. Mikrotiterplatten, Kugeln, Küvetten etc. aus gleichartigem Kunststoffmaterial.

100 µl Konjugatlösung (entsprechend 50 ng Konjugat in 0.1 molarem Bicarbonatpuffer pH 9.6) werden in

die Röhrchen einpipettiert und über 24 Stunden bei 37°C getrocknet. Die trocknen Röhrchen werden jeweils einmal mit 200 Microliter 10 mmol/l Phosphatpuffer pH 7.2 in isotoner Kochsalzlösung enthaltend 0.1 % PBS (phosphat buffered saline)/Tween 80 gewaschen und nach Absaugen der Waschlösung nochmals mit 200 Mikroliter destilliertem Wasser gespült und wieder abgesaugt. Die mit dem Konjugat beschichteten Röhrchen werden im Trockenschrank bei 37°C aufbewahrt.

3. Durchführung des Testes.

100 Mikroliter spezifische Standardlösung, enthaltend 0.1 µg bis 10 µg IgG (siehe Standardherstellung) in PBS/Tween 80-Puffer, werden in die wie oben beschrieben beschichteten Röhrchen einpipettiert und 1 Stunde bei 20°C inkubiert. Untersuchungen von biologischem Material, z.B. Patientenseren, werden, um in den Meßbereich der Standardkurve zu gelangen, je nach Titer 1:5 bis 1:2000 mit PBS/Tween 80 Puffer verdünnt und in gleichem Volumen eingesetzt. Der Inkubationsansatz wird abgesaugt und anschließend 2 mal mit 200 Microliter PBS/Tween 80-Puffer gewaschen und wieder abgesaugt. 100 µl (Antihuman IgG)-Immunglobulin (z.B. von der Ziege) markiert mit Peroxidase oder alkalischer Phosphatase werden in die Röhrchen einpipettiert und 1 Stunde bei konstanter Temperatur entweder bei 20°C, 25°C oder 37°C inkubiert. Anschließend wird nochmals 2 mal mit je 20 Microliter PBS/Tween-Puffer gewaschen. Bei Verwendung des Indikatorsystems konjugierte Peroxidase werden als Substrat o-Phenylendiamin und $H_2O_2$ in Citratpuffer 0,1 mol/l, pH 5.0, zugesetzt und 15 Minuten bei konstanter Temperatur inkubiert.

Zum Stoppen der Reaktion werden 50 µl 5 normale $H_2SO_4$ zugesetzt. Die Extinktion bei 492 nm ist proportional zur eingesetzten Immunglobulinmenge (s.Abb.1).

In Humanseren findet man Konzentrationen von 1 µg pro ml bis in den mg-Bereich.

Der Variationskoeffizient für die Bestimmung der spezifischen IgG's liegt im mittleren Bereich der Eichkurve bei 6.3 %.

## Gewinnung eines spezifischen humanen Immunglobulin-G-Standards.

1. Kopplung des Albumin-D-Ala-3-konjugats an Sepharose 4 b.

   Die Synthese erfolgt wie für den Test beschrieben. 10 ml Sepharosegel werden 5 mal mit 4 bis 5-fachem Überschuß an destilliertem Wasser gewaschen und dann in 10 ml Wasser suspendiert. Dazu werden 430 mg Bromcyan, gelöst in 8 ml Wasser, gegeben. Die Reaktion findet bei Raumtemperatur statt, der pH-Wert wird auf 11.0 durch Zugabe von 2 mol/l Natronlauge konstant gehalten. Nach 15-minütiger Reaktion unter leichtem Rühren wird das Gel auf einen Büchnertrichter abgesaugt und mit 60 ml 0.1 mol/l Natriumhydrogencarbonatlösung nachgewaschen. Die Sepharose wird in 7.5 ml 0.1 mol/l Natriumhydrogencarbonat, die 50 mg Albumin-D-Ala-3-konjugat enthalten, eingerührt. Die Koppelung erfolgt über Nacht bei 4°C unter leichtem Rühren. Nach der Reaktionszeit wird wieder auf der Filternutsche abgesaugt und das gekoppelte Gel jeweils 2 mal mit 30 bis 40 ml Wasser, dann 0.1 mol/l Natriumhydrogencarbonat, 0.2 mol/l Natriumacetatpuffer pH 4.5, 0.2 mol/l Natriumphosphatpuffer pH 7.2 und 0,01 Mol/l PBS pH 7.2 gewaschen. Das Affinitätsgel wird in PBS-Puffer pH 7.2 bei 4°C aufgehoben.

2. Durchführung der Affinitätschromatographie

Das Gel wird in eine passende Säule gefüllt und antikörperhaltiges Serum bzw. eine angereicherte Immunglobulinfraktion aufgegeben. Die beschickte Säule wird mit PBS-Puffer (ohne Tween) gewaschen, bis die Extinktion bei 280 nm im Eluat unter 0.03 absinkt (Verbrauch ca. die doppelte Menge Puffer wie Serum).
Die Elution erfolgt mit Essigsäure (0.1 - 1 mol/l). Der Antikörper wird mit den ersten Fraktionen der Essigsäure eluiert, die antikörperhaltigen Fraktionen werden vereinigt und neutralisiert. Die vereinigten Eluate werden gegen eine ausreichende Menge PBS dialysiert, konzentriert und anschließend auf Sephadex G 200 chromatographiert.

3. Charakterisierung der Antikörperfraktion.

Das gereinigte Immunglobulin wurde als symmetrischer Peak, vermessen bei 280 nm, von einer geeichten Sephadex-G-200-Säule eluiert. Das Molekulargewicht dieser Fraktion lag bei etwa 150.000. Eine weitere Absicherung des Molekulargewichts und der Homogenität erfolgt durch analytische Ultrazentrifugation, vermessen mit der UV-Optik bei 280 nm. Das Protein sedimentierte als einheitliche Bande. Unter Standardbedingungen ergab sich eine $S_{20w}$ von 7.8. Auf der Celluloseacetaleketrophorese ergab sich eine einheitliche Bande im Bereich der Gammaglobuline. In der Immunelektrophorese war bei Verwendung eines polyvalenten Anti-Serums eine Präzipitationslinie im Immunglobulin-G-Bereich nachzuweisen.

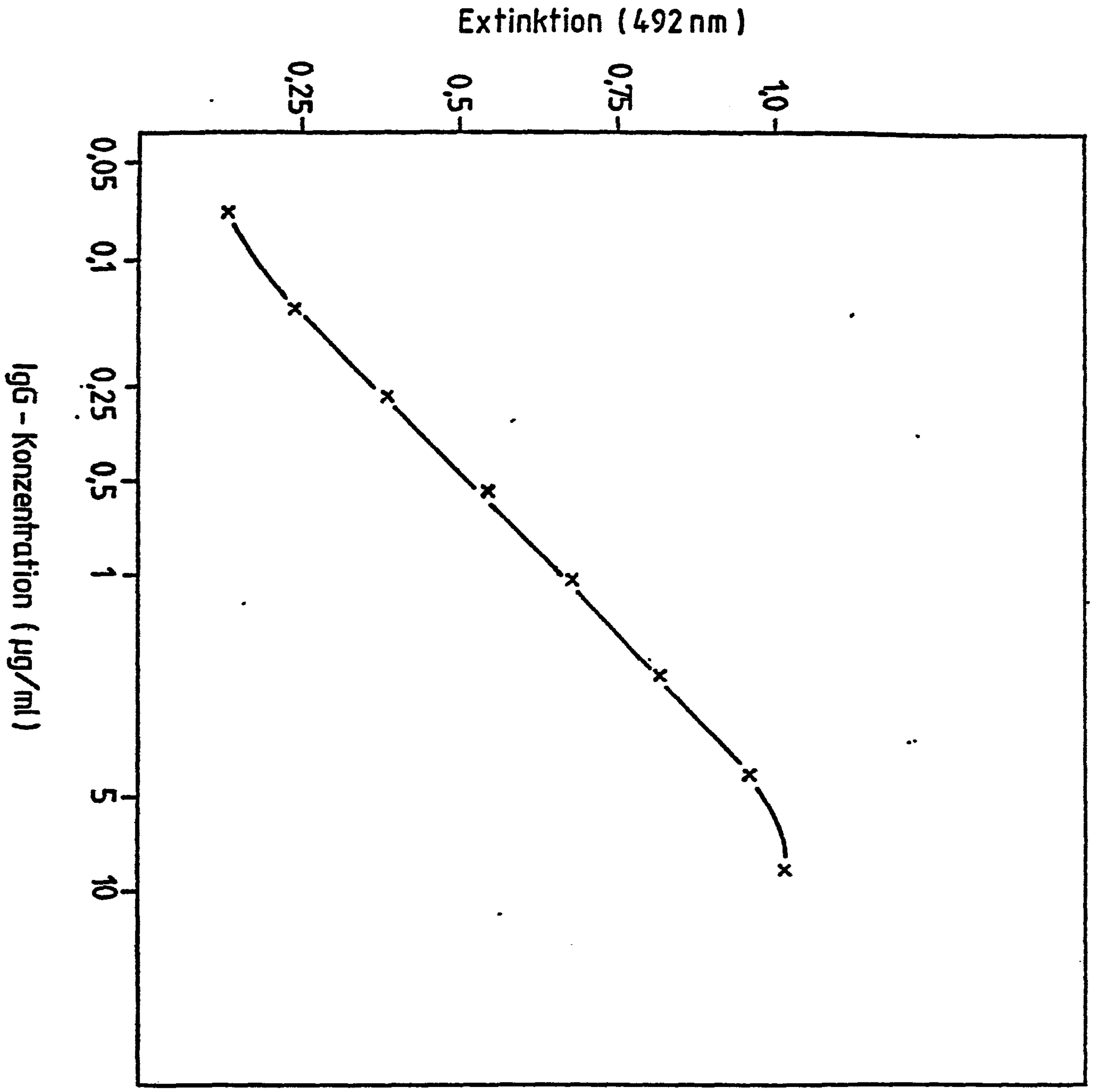


Abb.1

Eichkurve zur quantitativen Bestimmung von spezifischem Immunglobulin G (IgG) gegen ein Pentapeptid R-D-Ala-D-Ala. Als Indikator wurde das Peroxidasesystem verwendet. Die Extinktion bei 492 nm ist gegen die Konzentration von gereinigtem, spezifischem IgG aufgetragen.

## P a t e n t a n s p r ü c h e

1. Antikörper aus der Klasse der Immunglobuline G gegen das Peptiduntereinheit-Pentapeptid L-Ala-D-Glu(L-Lys-D-Ala-D-Ala) des Peptidoglycans in biologischem Untersuchungsmaterial sowie gegen diese Peptidoglycane enthaltende bakterielle Zellwände, gekennzeichnet durch
   a) spezifische Bindung an Pentapeptide der allgemeinen Formel

$$R - A_1 - D - Ala \qquad (I)$$

   worin
   R ein Aminosäureanteil mit 1 - 3, vorzugsweise 3 Aminosäuren aus der Gruppe Gly, L-Ala, D-Glu, L-Lys und
   $A_1$ eine der Aminosäuren D-Ala, D-Ser, D-Val oder D-Leu bedeuten, oder
   b) spezifische Bindung an Pentapeptide der allgemeinen Formel I enthaltende isolierte bakterielle Zellwände oder
   c) spezifische Bindung an Pentapeptide der allgemeinen Formel I enthaltende grampositive Bakterien.

2. Verfahren zur Herstellung der Antikörper gegen das Peptiduntereinheit-Pentapeptid L-Ala-D-Glu(L-Lys-D-Ala-D-Ala) des Peptidoglycans sowie gegen diese Pentapeptide enthaltende Zellwände, dadurch gekennzeichnet, daß ein Pentapeptid der allgemeinen Formel I gemäß Anspruch 1 nach an sich bekannten Methoden an eine feste Phase gekoppelt und in dieser Form zur selektiven Bindung und anschließender selektiven Elution des spezifischen Antikörpers aus der Klasse der Immunglobuline G aus einer durch Fällungsreaktion angereicherten Immunglobulinfraktion aus menschlichem Plasma verwendet wird.

3. Verfahren zur quantitativen Bestimmung von Antikörpern aus der Klasse der Immunglobuline G gegen das Peptiduntereinheit-Pentapeptid L-Ala-D-Glu(L-Lys-D-Ala-D-Ala) des Peptidoglycans in biologischem Untersuchungsmaterial nach an sich bekannten immunochemischen Verfahren, dadurch gekennzeichnet, daß man als Antigen eine Verbindung der allgemeinen Formel

$$R - A_1 - D - Ala \qquad (I)$$

worin

R ein Aminosäureanteil mit 1 - 3, vorzugsweise 3 Aminosäuren aus der Gruppe Gly, L-Ala, D-Glu, L-Lys und

$A_1$ eine der Aminosäuren D-Ala, D-Ser, D-Val oder D-Leu bedeutet, verwendet.

4. Verfahren nach Anspruch 3, dadurch gekennzeichnet, daß die quantitative Bestimmung von Antikörpern in einem Doppel-Antikörper-Radioimmun-Assay durchgeführt wird.

5. Verfahren nach Anspruch 3, dadurch gekennzeichnet, daß die quantitative Bestimmung von Antikörpern in einem Doppel-Antikörper-Enzymimmun-Assay durchgeführt wird.

6. Verfahren nach Anspruch 3, dadurch gekennzeichnet, daß die quantitative Bestimmung von Antikörpern mittels einer nephelometrischen Methode durchgeführt wird.

7. Verfahren nach Anspruch 3, dadurch gekennzeichnet, daß die quantitative Bestimmung von Antikörpern in einem Festphasen-Sandwich-Radioimmun-Assay durchgeführt wird.

8. Verfahren nach Anspruch 3, dadurch gekennzeichnet, daß die quantitative Bestimmung von Antikörpern in einem Festphasen-Enzymimmun-Assay durchgeführt wird.

9. Verfahren nach Anspruch 3, dadurch gekennzeichnet, daß die quantitative Bestimmung von Antikörpern in einem Festphasen-Fluoreszenz-Immun-Assay durchgeführt wird.

10. Verfahren nach Anspruch 3, dadurch gekennzeichnet, daß die quantitative Bestimmung von Antikörpern mittels eines Latex-Agglutinationstests durchgeführt wird.

11. Verwendung des nach Anspruch 2 hergestellten spezifischen Immunglobulins der Klasse G als Standard zur quantitativen Messung oder als Positivkontrolle für die in den Ansprüchen 3 - 10 aufgeführten Verfahren.